# EUROPEAN PATENT SPECIFICATION

(11) **EP 4 215 145 B1**
(45) Date of publication and mention of the grant of the patent: **20.05.2026**
(21) Application number: 23152493.5
(22) Date of filing: 19.01.2023
(51) Int. Cl.: A61B 18/14

(54) **SYSTEMS AND METHODS FOR C-SHAPED SPINES FORMING A SPHERICAL BASKET FOR IMPROVED TISSUE CONTACT AND CURRENT DELIVERY**
SYSTEME UND VERFAHREN FÜR C-FÖRMIGE DORNE ZUR FORMUNG EINES KUGELFÖRMIGEN KORBS FÜR VERBESSERTEN GEWEBEKONTAKT UND VERBESSERTE STROMABGABE
SYSTÈMES ET PROCÉDÉS POUR DES COLONNES EN FORME DE C FORMANT UN PANIER SPHÉRIQUE POUR UN CONTACT TISSULAIRE ET UNE DISTRIBUTION DE COURANT AMÉLIORÉS

(30) Priority: 20.01.2022 US 202263301107 P; 14.12.2022 US 202218065876
(43) Date of publication of application: 26.07.2023
(73) Proprietor: Biosense Webster (Israel) Ltd., Yokneam 2066717 (IL)
(72) Inventor: OKARSKI, Kevin Mark, Irvine 92618 (US); DATTA, Keshava, Irvine 92618 (US); BAH, Abubakarr, Irvine 92618 (US); NGUYEN, Thanh, Irvine 92618 (US)
(74) Representative: Carpmaels & Ransford LLP

(56) References cited:
- US-A- 5 893 847
- US-A1- 2013 090 651
- US-A1- 2014 288 552
- US-A1- 2017 035 496
- US-A1- 2017 319 140
- US-A1- 2021 369 339

## Description

### FIELD

The present invention relates generally to medical devices, and in particular catheters with electrodes, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain rare drawbacks due to operator's skill, such as heightened risk of thermal cell injury which can lead to tissue charring, burning, steam pop, phrenic nerve palsy, pulmonary vein stenosis, and esophageal fistula. Cryoablation is an alternative approach to RF ablation that generally reduces thermal risks associated with RF ablation but may present tissue damage due to the very low temperature nature of such devices. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Adhering the electrodes to the spines and then forming a spherical basket from the spines can be a difficult task, increasing the manufacturing time and cost and the chances that the electrode fails due to an improper bond or misalignment. What is needed, therefore, are devices and methods of forming an improved basket assembly that can help to reduce the time required for manufacturing the basket assembly and alternative basket assembly geometries in general.

US 2021/0369339 A1 provides an apparatus that includes an end effector having loop members with electrodes thereon and is usable with catheter-based systems to measure or provide electrical signals. The end effector can include three loop members that are noncoplanar when expanded unconstrained that become contiguous to a planar surface when the loop members are deflected against the surface, a mechanical linkage that joins the loop members at a distal vertex of the end effector, electrodes having surface treatment to enhance surface roughness of the electrodes, twisted pair electrode wires, a bonded spine cover, and/ or any combination thereof. US 2017/0319140 Al provides a catheter having a basket shaped electrode assembly with a high electrode density. The basket-shaped electrode assembly may have a plurality of spines, such as up to twelve, each with a plurality of electrodes, such as up to sixteen. The distal ends of the plurality of spines are joined at a distal hub, all of which are fashioned from a single piece of superelastic material.

### SUMMARY OF THE INVENTION

The invention is defined in independent claims 1 and 14. Advantageous embodiments of the invention are defined in the dependent claims.

### SUMMARY OF THE DISCLOSURE

Various embodiments of a medical probe and related methods are described and illustrated. The medical probe may include a tubular shaft including a proximal end and a distal end and extending along a longitudinal axis. The medical probe can include an expandable basket assembly proximate the distal end of the tubular shaft. The basket assembly can include two or more C-shaped spines converging at a central spine intersection configured to permit a bending of the C-shaped spines. Each C-shaped spine can include a respective end connected to the distal end of the tubular shaft. The central spine intersection can be positioned on the longitudinal axis at a distal end of the basket assembly. The basket assembly can further include one or more electrodes coupled to each of the C-shaped spines. Each electrode can define a lumen through the electrode so that the C-shaped spine can extend through the lumen of each of the one or more electrodes.

The disclosed technology can include an example method of constructing a medical probe. The method may include cutting a planar sheet of material to form a plurality of C-shaped spines including a central spine intersection; inserting each C-shaped spine into a lumen of at least one electrode; and fitting ends of the plurality of C-shaped spines to a tubular shaft sized to traverse vasculature such that the central spine intersection is positioned at a distal end of the medical probe and respective C-shaped spines are movable from a tubular configuration to a bowed configuration.

### BRIEF DESCRIPTION OF THE DRAWINGS

FIG. 1 is a schematic pictorial illustration of a medical system including a medical probe whose distal end includes a basket assembly with electrodes, in accordance with an embodiment of the present invention;
FIG. 2A is a schematic pictorial illustration showing a perspective view of a medical probe in an expanded form, in accordance with an embodiment of the present invention;
FIG. 2B is a schematic pictorial illustration showing a side view of a medical probe in a collapsed form, , in accordance with embodiments of the present invention;
FIG. 2C is a schematic pictorial illustration showing an exploded side view of a medical probe, in accordance with an embodiment of the present invention;
FIGs. 3A and 3B are schematic pictorial illustrations showing a profile outline of a basket assembly of a given medical device, in accordance with embodiments of the present invention;
FIG. 4 is a schematic pictorial illustration showing a side view of C-shaped spines forming a basket assembly, in accordance with an embodiment of the present invention;
FIGs. 5A and 5B are schematic pictorial illustrations of a method of forming a basket assembly, in accordance with an embodiment of the present invention;
FIGs. 6A and 6B are schematic pictorial illustrations of top-down views of basket assembly 38, showing various examples of central intersection, in accordance with an embodiment of the present invention;
FIGs. 7A through 7J are schematic pictorial illustrations showing a perspective view of various example electrodes, in accordance with embodiments of the present invention;
FIGs. 8A and 8B are schematic pictorial illustrations showing various insulative jackets of a given medical device, in accordance with embodiments of the present invention;
FIGs. 9A and 9B are schematic pictorial illustrations showing cross-sectional views of a given wire of a medical probe, in accordance with an embodiment of the present invention;
FIGs. 10A through 10C are schematic pictorial illustrations of cutting patterns for C-shaped spines from a planar sheet of material, in accordance with an embodiment of the present invention;
FIGs. 11A and 11B are schematic pictorial illustrations of a method of cutting a plurality of C-shaped spines including one or more cutouts at a central spine intersection from a planar sheet of material, in accordance with an embodiment of the present invention; and
FIG. 12 is a flowchart illustrating another method of assembling a basket assembly, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, methods or uses and devices which utilize end effectors including electrodes affixed to spines. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1.

To deliver pulsed field ablation (PFA) in an IRE (irreversible electroporation) procedure, electrodes should contact the tissue being ablated with a sufficiently large surface area. As described hereinbelow, the medical probe includes a tubular shaft including proximal and distal ends, and a basket assembly at the distal end of the tubular shaft. The basket assembly includes two or more C-shaped spines converging at a central intersection and including one or more electrodes coupled to each of the spines. The C-shaped spines can bend to form an approximately spherical or oblate-spheroid basket assembly.

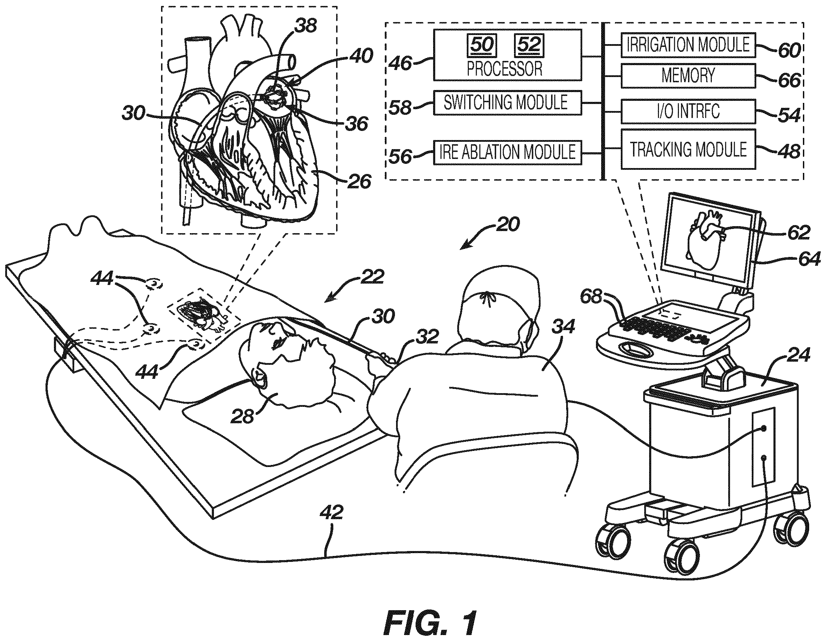

FIG. 1 is a schematic, pictorial illustration of a medical system 20 including a medical probe 22 and a control console 24, in accordance with an embodiment of the present invention. Medical system 20 may be based, for example, on the CARTO^{®} system, produced by Biosense Webster Inc. of 31 Technology Drive, Suite 200, Irvine, CA 92618 USA. In embodiments described hereinbelow, medical probe 22 can be used for diagnostic or therapeutic treatment, such as for performing ablation procedures in a heart 26 of a patient 28. Alternatively, medical probe 22 may be used, mutatis mutandis, for other therapeutic and/or diagnostic purposes in the heart or in other body organs.

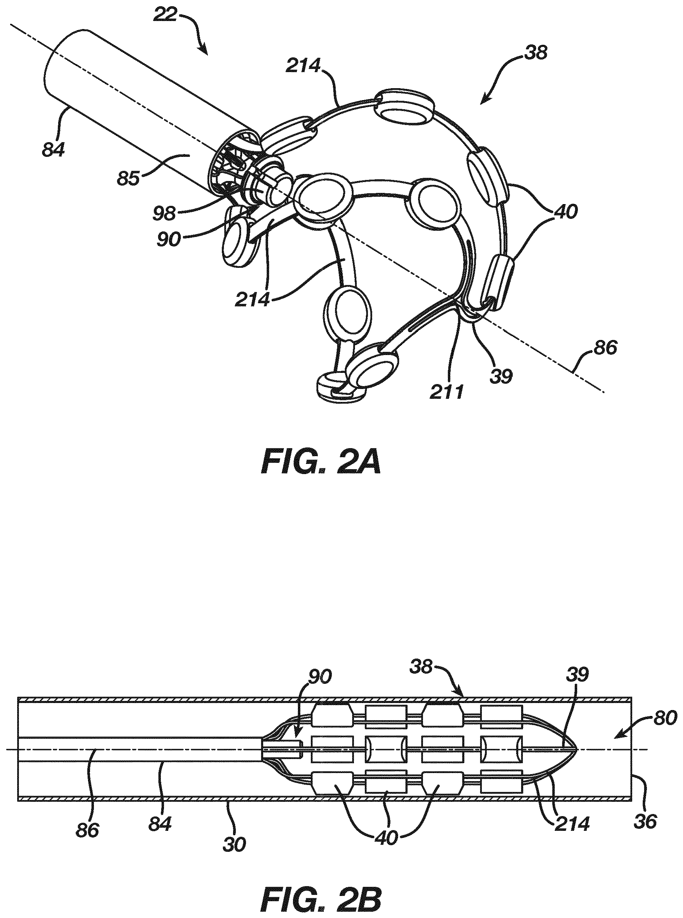

Medical probe 22 includes a flexible insertion tube 30 and a handle 32 coupled to a proximal end of the tubular shaft. During a medical procedure, a medical professional 34 can insert probe 22 through the vascular system of patient 28 so that a distal end 36 of the medical probe enters a body cavity such as a chamber of heart 26. Upon distal end 36 entering the chamber of heart 26, medical professional 34 can deploy a basket assembly 38 approximate a distal end 36 of the medical probe 22. Basket assembly 38 can include a plurality of electrodes 40 affixed to a plurality of spines 214, as described in the description referencing FIGs. 2A and 2B hereinbelow. To start performing a medical procedure such as irreversible electroporation (IRE) ablation, medical professional 34 can manipulate handle 32 to position distal end 36 so that electrodes 40 engage cardiac tissue at a desired location or locations. Upon positioning the distal end 36 so that electrodes 40 engages cardiac tissue, the medical professional 34 can activate the medical probe 22 such that electrical pulses are delivered by the electrodes 40 to perform the IRE ablation.

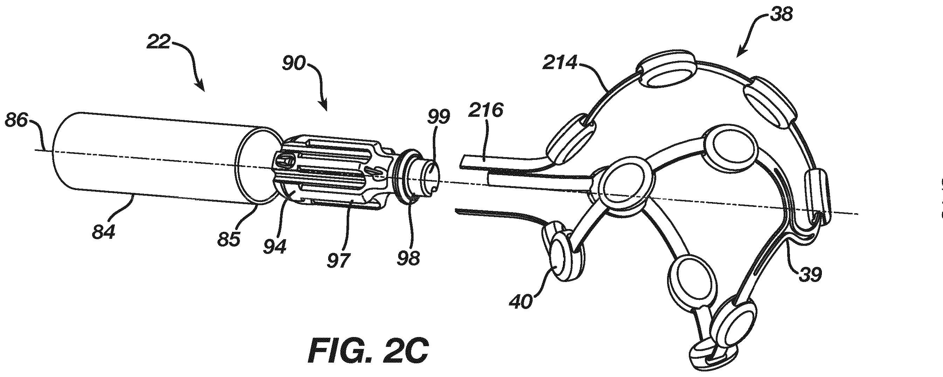

The medical probe 22 can include a guide sheath and a therapeutic catheter, wherein the guide sheath includes the flexible insertion tube 30 and the handle 32 and the therapeutic catheter includes the basket assembly 38, electrodes 40, and a tubular shaft 84 (see FIGs. 2 through 4). The therapeutic catheter is translated through the guide sheath so that the basket assembly 38 is positioned in the heart 26. The distal end 36 of the medical probe 22 corresponds to a distal end of the guide sheath when the basket assembly 38 is contained within the flexible insertion tube 30, and the distal end 36 of the medical probe 22 corresponds to a distal end of the basket assembly 38 when the basket assembly 38 is extended from the distal end of the guide sheath. The medical probe 22 can be alternatively configured to include a second handle on the therapeutic catheter and other features as understood by a person skilled in the pertinent art.

In the configuration shown in FIG. 1, control console 24 is connected, by a cable 42, to body surface electrodes, which typically include adhesive skin patches 44 that are affixed to patient 28. Control console 24 includes a processor 46 that, in conjunction with a tracking module 48, determines location coordinates of distal end 36 inside heart 26. Location coordinates can be determined based on electromagnetic position sensor output signals provided from the distal portion of the catheter when in the presence of a generated magnetic field. Location coordinates can additionally, or alternatively be based on impedances and/or currents measured between adhesive skin patches 44 and electrodes 40 that are affixed to basket assembly 38. In addition to being used as location sensors during a medical procedure, electrodes 40 may perform other tasks such as ablating tissue in the heart.

As described hereinabove, in conjunction with tracking module 48, processor 46 may determine location coordinates of distal end 36 inside heart 26 based on impedances and/or currents measured between adhesive skin patches 44 and electrodes 40. Such a determination is typically after a calibration process relating the impedances or currents to known locations of the distal end has been performed. While embodiments presented herein describe electrodes 40 that are preferably configured to deliver IRE ablation energy to tissue in heart 26, configuring electrodes 40 to deliver any other type of ablation energy to tissue in any body cavity is possible. Furthermore, although described in the context of being electrodes 40 that are configured to deliver IRE ablation energy to tissue in the heart 26, one skilled in the art will appreciate that the disclosed technology can be applicable to electrodes used for mapping and/or determining various characteristics of an organ or other part of the patient's 28 body.

Processor 46 may include real-time noise reduction circuitry 50 typically configured as a field programmable gate array (FPGA), followed by an analog-to-digital (A/D) signal conversion integrated circuit 52. The processor can be programmed to perform one or more algorithms and uses circuitry 50 and circuit 52 as well as features of modules to enable the medical professional 34 to perform the IRE ablation procedure.

Control console 24 also includes an input/output (I/O) communications interface 54 that enables control console 24 to transfer signals from, and/or transfer signals to electrodes 40 and adhesive skin patches 44. In the configuration shown in FIG. 1, control console 24 additionally includes an IRE ablation module 56 and a switching module 58.

IRE ablation module 56 is configured to generate IRE pulses including peak power in the range of tens of kilowatts. In some examples, the electrodes 40 are configured to deliver electrical pulses including a peak voltage of at least 900 volts (V) to approximately 2000V. The medical system 20 performs IRE ablation by delivering IRE pulses to electrodes 40. Preferably, the medical system 20 delivers biphasic pulses between electrodes 40 on the spine. Additionally, or alternatively, the medical system 20 delivers monophasic pulses between at least one of the electrodes 40 and a skin patch.

The system 20 may supply irrigation fluid (e.g., a saline solution) to distal end 36 and to the electrodes 40 via a channel (not shown) in tubular shaft 84 (see FIGs. 2A through 2C). Irrigation is sometimes utilized to prevent stagnation of blood flow, pooling of blood or clot formation. Additionally, or alternatively, irrigation fluid can be supplied through the flexible insertion tube 30. Control console 24 includes an irrigation module 60 to monitor and control irrigation parameters, such as the pressure and the temperature of the irrigation fluid. It is noted that while the preference for the exemplary embodiments of the medical probe is for IRE or PFA, it is possible to also use the medical probe separately only for RF ablation (unipolar mode with an external grounding electrode or bipolar mode) or in combination with IRE and RF ablations sequentially (certain electrodes in IRE mode and other electrodes in RF mode) or simultaneously (groups of electrodes in IRE mode and other electrodes in RF mode).

Based on signals received from electrodes 40 and/or adhesive skin patches 44, processor 46 can generate an electroanatomical map 62 that shows the location of distal end 36 in the patient's body. During the procedure, processor 46 can present map 62 to medical professional 34 on a display 64, and store data representing the electroanatomical map in a memory 66. Memory 66 may include any suitable volatile and/or non-volatile memory, such as random-access memory or a hard disk drive.

In some embodiments, medical professional 34 can manipulate map 62 using one or more input devices 68. In alternative embodiments, display 64 may include a touchscreen that can be configured to accept inputs from medical professional 34, in addition to presenting map 62.

FIG. 2A is a schematic pictorial illustration showing a perspective view of a medical probe 22 including a basket assembly 38 in an expanded form when unconstrained, such as by being advanced out of an insertion tube lumen 80 at a distal end 36 of an insertion tube 30. The medical probe 22 illustrated in FIG. 2A lacks the guide sheath illustrated in FIG. 1. FIG. 2B shows the basket assembly a collapsed form within insertion tube 30 of the guide sheath. In the expanded form (FIG. 2A), spines 214 bow radially outwardly and in the collapsed form (FIG. 2B) the spines are arranged generally along a longitudinal axis 86 of insertion tube 30.

As shown in FIG. 2A, basket assembly 38 includes a plurality of flexible C-shaped spines 214 that are formed at the end of a tubular shaft 84 and are connected at a proximal end to the tubular shaft 84. During a medical procedure, medical professional 34 can deploy basket assembly 38 by extending tubular shaft 84 from insertion tube 30 causing basket assembly 38 to exit insertion tube 30 and transition to the expanded form. Spines 214 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

As shown in FIG. 2A, the plurality of flexible C-shaped spines 214 converge at a central spine intersection 211. In some examples central spine intersection 211 can include one or more cutouts 212 that allow for bending of the spines 214 when each spine respective attachment end 216 is connected to the spine retention hub 90, described in more detail below. It is noted that the cutouts (in various configurations described and illustrated in the specification allows for a much smaller form factor when undeployed (or undergoing a retraction into a delivery sheath) without buckling or plastic deformation.

The basket assembly 38 can be easier to manufacture compared to a basket assembly including multiple C-shaped spines assembled together by attaching both ends of the C-shaped spines to a tubular shaft (e.g., a pusher tube) to form a spherical basket. The basket assembly 38 can also include electrodes 40 over the spines that are easier to manufacture compared to a basket assembly including electrodes adhered to the spines and then forming a spherical basket. Due to the small size of the spines and the electrodes, adhering electrodes to the spines can be a difficult task, increasing the manufacturing time and cost and the chances that the electrode fails due to an improper bond or misalignment.

In embodiments described herein, one or more electrodes 40 positioned on spines 214 of basket assembly 38 can be configured to deliver ablation energy (RF and/or IRE) to tissue in heart 26. Additionally, or alternatively, the electrodes can also be used to determine the location of basket assembly 38 and/or to measure a physiological property such as local surface electrical potentials at respective locations on tissue in heart 26. The electrodes 40 can be biased such that a greater portion of the one or more electrodes 40 face outwardly from basket assembly 38 such that the one or more electrodes 40 deliver a greater amount of electrical energy outwardly away from the basket assembly 38 (i.e., toward the heart 26 tissue) than inwardly.

Examples of materials ideally suited for forming electrodes 40 include gold, platinum and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 26.

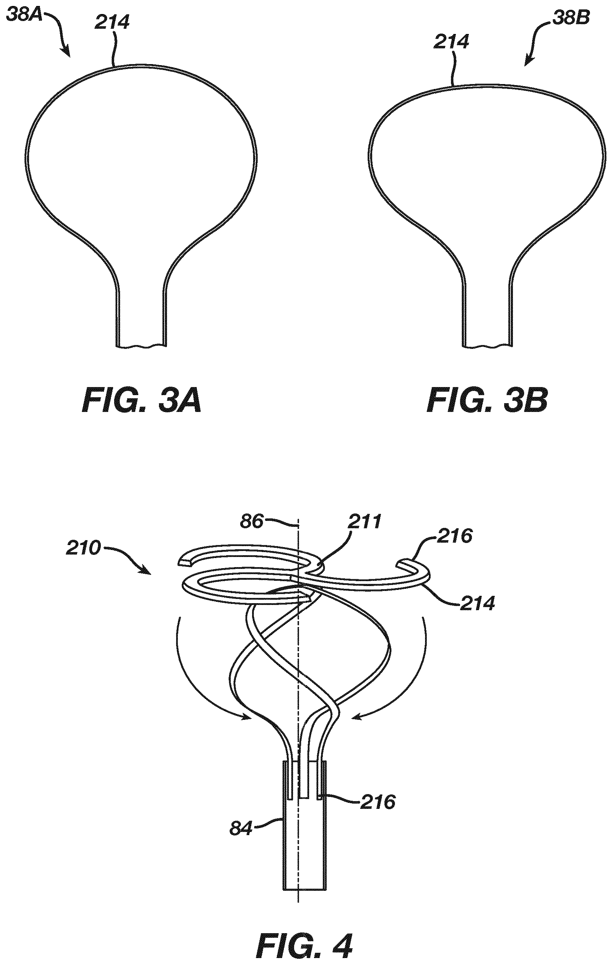

Turning to FIG. 2C, basket assembly 38 includes a single unitary structure that includes a plurality of C-shaped spines 214 formed from a planar sheet of material 210 (shown more clearly in FIGs. 3 and 4A). The medical probe 22 can include a spine retention hub 90 disposed proximate the distal end 85 of the tubular shaft 84. The spine retention hub 90 can be inserted into the tubular shaft 84 and attached to the tubular shaft 84. Spine retention hub 90 can include a cylindrical member 94 including a plurality of relief lands 96, multiple irrigation openings 98, and at least one spine retention hub electrode 99. Relief lands 96 can be disposed on the outer surface of cylindrical member 94 and configured to allow a portion of each spine 214, such as each spine attachment end 216, to be fitted into a respective relief land 96. The attachment end 216 can be a generally linear end of the spine 214. The attachment end 216 can be configured to extend distally from the spine retention hub 90 such that the basket assembly 38 is positioned distally from the spine retention hub 90 and, consequently, distally from the tubular shaft 84. In this way, the spine 214 can be configured to position the basket assembly 38 distally from the distal end of the tubular shaft 84 and distal from the distal end of the insertion tube 30 when the basket assembly is deployed.

As described supra, control console 24 includes irrigation module 60 that delivers irrigation fluid to distal end 36. The multiple irrigation openings 98 can be angled to spray or otherwise disperse of the irrigation fluid to either a given electrode 40 or to tissue in heart 26. Since electrodes 40 do not include irrigation openings that deliver irrigation fluid, the configuration described hereinabove enables heat to be transferred from the tissue (i.e., during an ablation procedure) to the portion of the electrodes on the inner side of the spines 214, and the electrodes 40 can be cooled by aiming the irrigation fluid, via irrigation openings 98, at the portion of the electrodes 40 on the inner side of the spines 214. Spine retention hub electrode 99 disposed at a distal end of spine retention hub 90 can be used in combination with electrodes 40 on the spines 214, or alternatively, can be used independently from electrodes 40 for reference mapping or ablation.

FIGs. 3A and 3B are schematic pictorial illustrations showing a profile outline of spines 214 of a basket assembly 38A, 38B of a given medical device 22, in accordance with embodiments of the present invention. To illustrate, the basket assembly can have a profile as shown in FIG. 3A that is approximately circular to form an approximately spherical shape when basket assembly 38A is in the expanded form. As another example, the basket assembly can have a profile as shown in FIG. 3B that is an approximately elliptical shape to form an approximately oblate-spheroid shape when basket assembly 38B is in the expanded form. Although not every variation of shape is shown or described herein, one skilled in the art will appreciate that spines 214 can be further configured to form other various shapes as would be suitable for the particular application.

By including spines 214 configured to form various shapes when in the expanded form, basket assembly 38 can be configured to position the various electrodes 40 attached to spines 214 at various locations, with each location being nearer or farther from the distal end of tubular shaft 84. For example, electrode 40 attached to spine 214 illustrated in FIG. 3A near the middle of spine 214 would be farther from the distal end of tubular shaft 84 than spine 214 illustrated in FIG. 3B when basket assembly 38 is in the expanded form. In addition, each spines 214 may have an elliptical (e.g., circular) or rectangular (that may appear to be flat) cross-section, and include a flexible, resilient material (e.g., a shape-memory alloy such as nickel-titanium (also known as Nitinol), cobalt chromium, or any other suitable material).

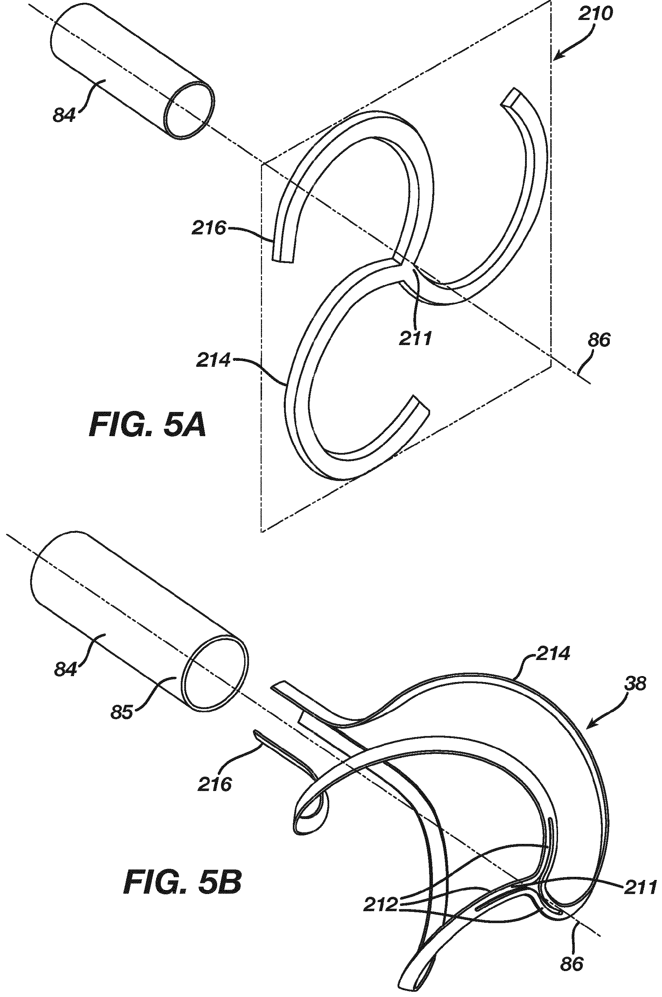

FIGs. 4, 5A and 5B are schematic pictorial illustrations showing views of spines 214 forming basket assembly 38. FIG. 4 provides one example of how planar sheet of material 210 may be assembled together with tubular shaft 84 whereby each spine 214 bends or curves when respective attachment ends 216 are connect to spine retention hub 90. As shown in FIG. 5A, the C-shaped spines 214 can be formed from a single sheet of planar material 210 to form a plurality of semi-circular shapes connected at a central point when laid flat. In other words, spines 214 can be formed from the single sheet of planar material such that the spines 214 converge toward a central intersection 211. Central intersection 211 can be a solid piece of material (as shown in FIG. 5A) or include one or more cutouts 212 (as shown in FIG. 5B). Basket assembly 38 can include a number of spines 214 ranging from about two to about five spines from a single sheet of planar material 210.

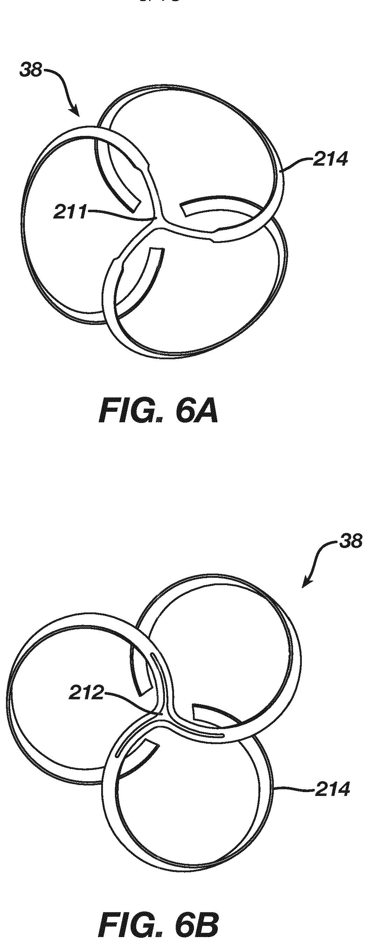

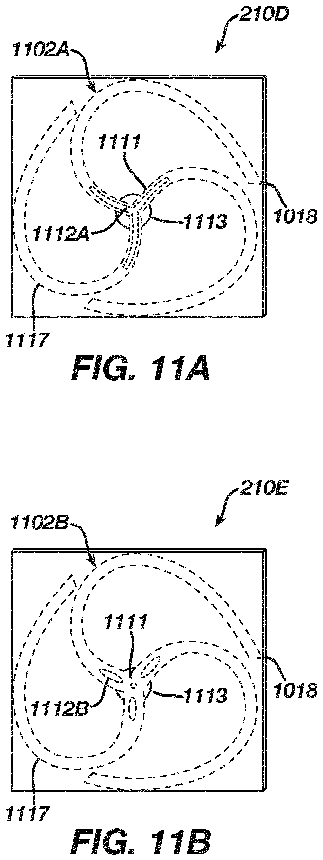

FIGs. 6A and 6B are schematic pictorial illustrations of top-down views of spines 214 of the basket assembly 38, showing various examples of central intersection 211 including no cutouts (FIG. 6A) or one or more cutouts 212 (FIG. 6B). As shown, intersection 211 can include a single discrete cutout 212. Alternatively, intersection 211 can include two or more cutouts, as provided as an example in FIG. 11B, discussed in more detail below. The one or more cutouts 212 can include a variety of patterns, such as centrosymmetric (i.e., symmetric with respect to a central point), and equiangular (i.e., including equal angles) to allow for equal bending among the spines 214 as well as disproportional and asymmetric to allow for unequal bending of spines 214 to alter structural stability. In certain instances, when basket assembly 38 includes an even number of C-shaped spines 214, the pattern of the one or more cutouts 212 can alter between every other spine. In some examples, one or more cutouts 212 can extend along a portion of each spine 214.

The spines 214 can be folded or otherwise bent such that each respective attachment end 216 of the spine 214 can be inserted into the distal end 85 of the tubular shaft 84 (as shown in FIG. 2B) and relief lands 96 of spine retention hub 90. Although not shown in FIGs. 5A through 6B, it will be appreciated that electrodes 40 can be attached to spines 214 before the spines are inserted into the tubular shaft 84 to form the basket assembly 38. As stated previously, the spines 214 can include a flexible, resilient material (e.g., a shape-memory alloy such as nickel-titanium, also known as Nitinol) that can enable the basket assembly 38 to transition to its expanded form (as shown in FIG. 2A) when the basket assembly 38 is deployed from tubular shaft 84. As will become apparent throughout this disclosure, spines 214 can be electrically isolated from electrode 40 to prevent arcing from electrode 40 to the respective spine 214.

As will be appreciated by one skilled in the art with the benefit of this disclosure, basket assembly 38 shown in FIGs. 2A through 2C including spines 214 formed from a planar sheet of material and converging at a central intersection is offered merely for illustrative purposes and the disclosed technology can be applicable to other configurations of basket assemblies 38. For example, the disclosed technology can be applicable to basket assemblies 38 formed from a single spine 214 or multiple spines 214 with each spine 214 being attached at both ends. In other examples, the basket assembly 38 can include a retention hub connecting the multiple spines 214 together at a distal end 39 of the basket assembly 38. In yet other examples, the basket assembly 38 can include a single spine 214 configured to form a spiral, multiple spines 214 configured to form a spiral, multiple spines 214 configured to form a tripod or multiple tripods, or any other shape of basket assembly 38. Thus, although FIGs. 2A through 2C illustrate a specific configuration of basket assembly 38, the disclosed technology should not be construed as so limited.

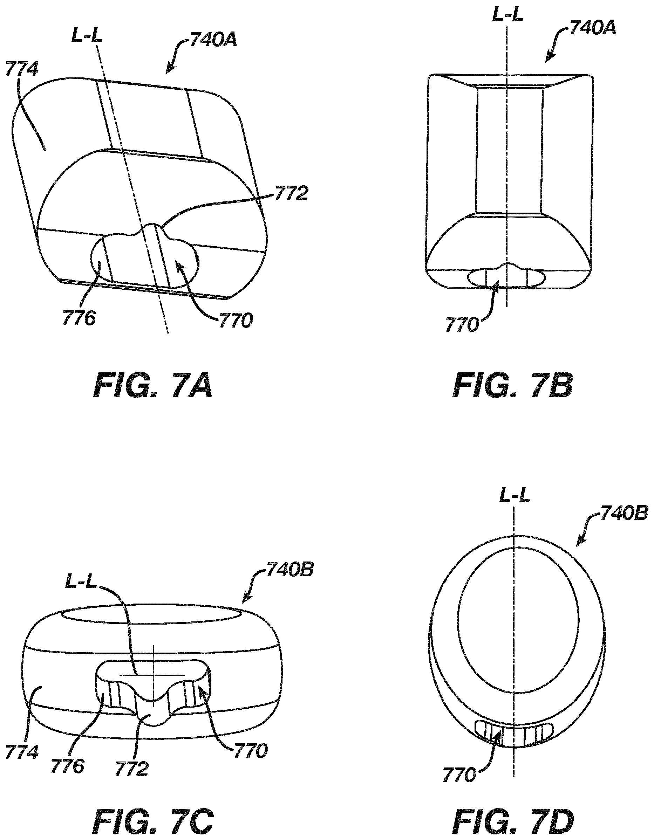

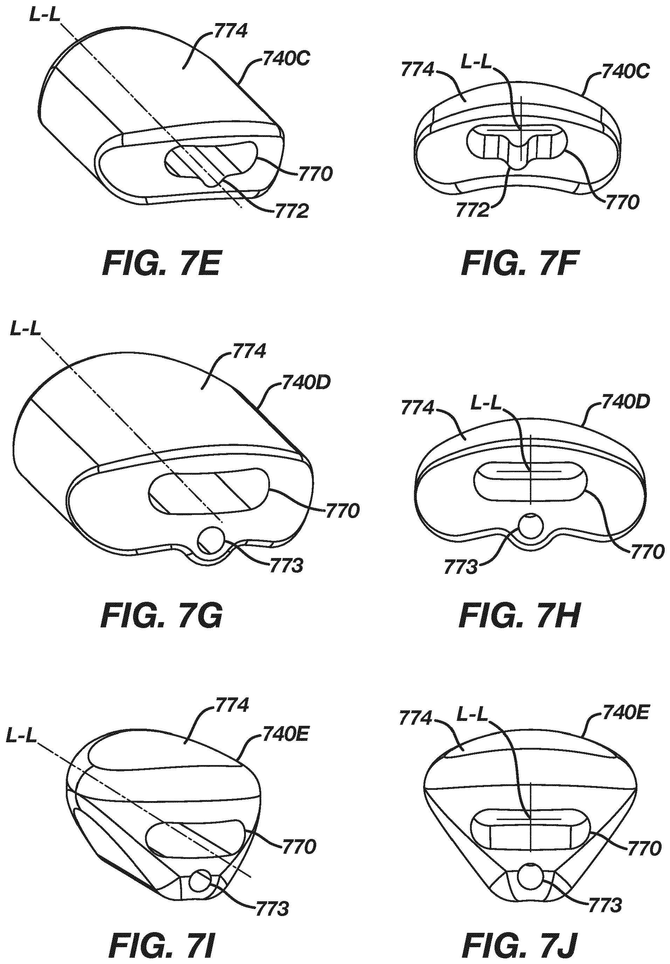

Referring back to FIGs. 2A through 2C, one or more electrodes 40 can be attached to spines 214 to form the basket assembly 38. In some examples, each electrode 40 can include electrically conductive material (e.g., gold, platinum and palladium (and their respective alloys)). Turning to FIGs. 7A through 7J, electrode 40 can have a variety of cross-sectional shapes, curvatures, lengths, lumen number and lumen shape as provided as examples in electrodes 740A-740E. The electrodes 740A-740E are offered to illustrate various configurations of electrodes 40 that can be used with the medical device 22 but should not be construed as limiting. One skilled in the art will appreciate that various other configurations of electrodes 40 can be used with the disclosed technology without departing from the scope of this disclosure.

Each electrode 740A-740E can have an outer surface 774 facing outwardly from electrode 740 and an inner surface 776 facing inwardly toward electrode 740 where at least one lumen 770 is formed through electrode 740. The lumen 770 can be sized and configured to receive a spine 214 such that spine 214 can pass through electrode 740. Lumen 770 can be a symmetric opening through electrode 740A-740E and can be disposed offset with respect to a longitudinal axis L-L of the respective electrode. In other examples, lumen 770 can pass through electrode 740 in a generally transverse direction with respect to the longitudinal axis L-L of the respective electrode. Furthermore, lumen 770 can be positioned in electrode 740 nearer a bottom surface, nearer a top surface, or nearer a middle of electrode 740 depending on the particular configuration. In FIGs. 7A, 7C, and 7E through 7J, the top surface (upper side) is oriented toward the top of the drawing, the bottom surface (lower side) is oriented toward the bottom of the drawing, and the middle is between the top surface and the bottom surface. In other words, each electrode 740A-740E can include a lumen 770 that is offset with respect to a centroid of the electrode740A-740E.

In addition, as shown in FIGs. 7A through 7F, electrodes 740A-740C can have a wire relief 772 forming a recess or depression in electrode 740 adjacent lumen 770 for one or more wires to pass through lumen 770 along with a respective spine 214. Relief 772 can be sized to provide room for a wire of electrode 740 to pass through electrode 740 such that electrode 740 can be in electrical communication with the control console 24.

Alternatively, or in addition thereto, wires can pass through a wire lumen 773 as shown in example electrodes 740D and 740E in FIGs. 7G through 7J. Although not depicted, electrodes 40 may include both a wire relief 772 adjacent lumen 770 and wire lumen 773. Such electrode may permit additional wires to pass through the electrode body.

As shown in FIGs. 7A-7J, the electrodes 740A-740E can include various shapes depending on the application. For example, as illustrated in FIGs. 7A and 7B, the electrode 740A can include a substantially rectangular cuboid shape with rounded edges. In other examples, the electrode 740B can include a substantially ovoid shape (as illustrated in FIGs. 7C and 7D), the electrode 740C, 740D can have a contoured shape including a convex side and a concave side (as illustrated in FIGs. 7E through 7H), or the electrode 740E can have a contoured shape including substantially more material proximate an upper side than a lower side of the electrode 740E (as illustrated in FIGs. 7I and 7J). As will be appreciated by one of skill in the art, the various example electrodes 740A-740E shown in FIGs. 7A-7J, and described herein, are offered for illustrative purposes and should not be construed as limiting.

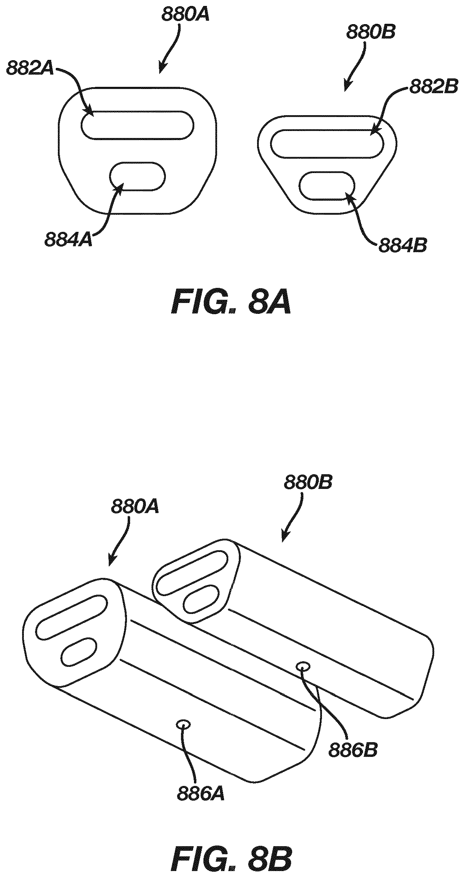

FIGs. 8A and 8B are schematic pictorial illustrations showing various insulative jackets 880A, 880B of a given medical device 22, in accordance with embodiments of the present invention. FIG. 8A is a front view while FIG. 8B is a perspective view of insulative jackets 880A, 880B. Insulative jackets 880A, 880B can be made from a biocompatible, electrically insulative material such as polyamide-polyether (Pebax) copolymers, polyethylene terephthalate (PET), urethanes, polyimide, parylene, silicone. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets 880A, 880B may also include one or more additives or fillers, such as, for example, polytetrafluoroethylene (PTFE), boron nitride, silicon nitride, silicon carbide, aluminum oxide, aluminum nitride, zinc oxide, and the like. Insulative jacket 880A, 880B can help to insulate a spine 214 and/or wires passing through insulative jacket 880A, 880B from electrode 40 to prevent arcing from electrode 40 to the spine 214 and/or mechanical abrasion of wires passing through insulative jacket 880A, 880B.

As illustrated in FIGs. 8A and 8B, insulative jackets 880A, 880B, can include a cross-sectional shape that is substantially trapezoidal. The insulative jacket may consist of a single lumen or multi-lumen configuration. Multi-lumen jackets may be configured such that the alloy frame and wires share a single lumen while the second lumen may be used for irrigation. The alloy frame and wires may occupy separate lumens, also, as described. The current embodiment does not utilize irrigated jackets. For these designs, the insulative jackets may be continuous (individual sleeves extending from proximal to distal end of each alloy frame strut), segmented (bridging between electrode gaps), or a combination of both. Furthermore, insulative jacket 880A, 880B can include a first lumen 882A, 882B and a second lumen 884A, 884B. First lumen 882A, 882B can be configured to receive spine 214 while second lumen 884A, 884B can be configured to receive a wire, or vice-versa. In other examples, first lumen 882A, 882B and second lumen 884A, 884B can each be configured to receive one or more wires that can be connected to one or more electrodes 40. Furthermore, as illustrated in FIG. 8B, insulative jacket 880A, 880B can include an aperture 886A, 886B through which a wire can be electrically connected to electrode 40. Although illustrated in FIG. 8B as being proximate a bottom of insulative jacket 880A, 880B, aperture 886A, 886B can be positioned proximate a top or side of insulative jacket 880A, 880B. Furthermore, insulative jacket 880A, 880B can include multiple apertures 886A, 886B with each aperture being disposed on the same side of insulative jacket (i.e., top, bottom, left, right) or on different sides of the insulative jacket depending on the application.

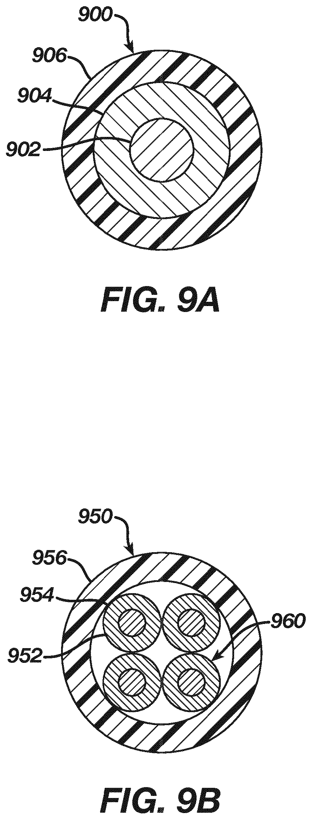

FIGs. 9A and 9B are schematic pictorial illustrations showing cross-sectional views of a given wire 900, 950 that can be connected to a given electrode 40, in accordance with an embodiment of the present invention. FIG. 9A illustrates a solid core wire 900. FIG. 9B illustrates a stranded wire 950. Each wire 900, 950 can extend through at least a portion of tubular shaft 84 and tubular shaft 84. Solid core wire 900 can include an electrically conductive core material 902 and an electrically conductive cover material 904 circumscribing electrically conductive core material 902. Likewise, stranded wire 950 can include strands each including an electrically conductive core material 952 and an electrically conductive cover material 954 circumscribing the electrically conductive core material 952. Each wire 900, 950 can include an insulative jacket 906 circumscribing the conductors. The wires 900, 950 can be configured to withstand a voltage difference of adjacent wires sufficient to deliver IRE pulses. Preferably, the wires 900, 950 can withstand at least 900V, and more preferably at least 1,800V between adjacent wires. To reduce likelihood of dielectric breakdown between conductors of adjacent wires, electrically conductive cover material 904, 954 can have a lower electrical conductivity compared to core material 902, 952.

Insulative jacket 906 can be configured to have a temperature rating between 150 and 200 degrees Centigrade so that the electrically insulative jacket 906 melts or degrades (e.g., chars and crumbles) during soldering of wire 900 to electrodes 40 (e.g., at a temperature of 300 degrees Centigrade) and therefore insulative jacket 906 of wire 900 does not need to be mechanically stripped. In other examples, insulative jacket 906 can have a temperature rating greater than 200 degrees Centigrade to prevent electrically insulating material 906 melting or degrading (e.g., charring and crumbling) during manufacture of medical probe 22 and/or during use. Insulative jacket 906 can be mechanically stripped from wire 900 prior to wires 900 being electrically connected to electrodes 40.

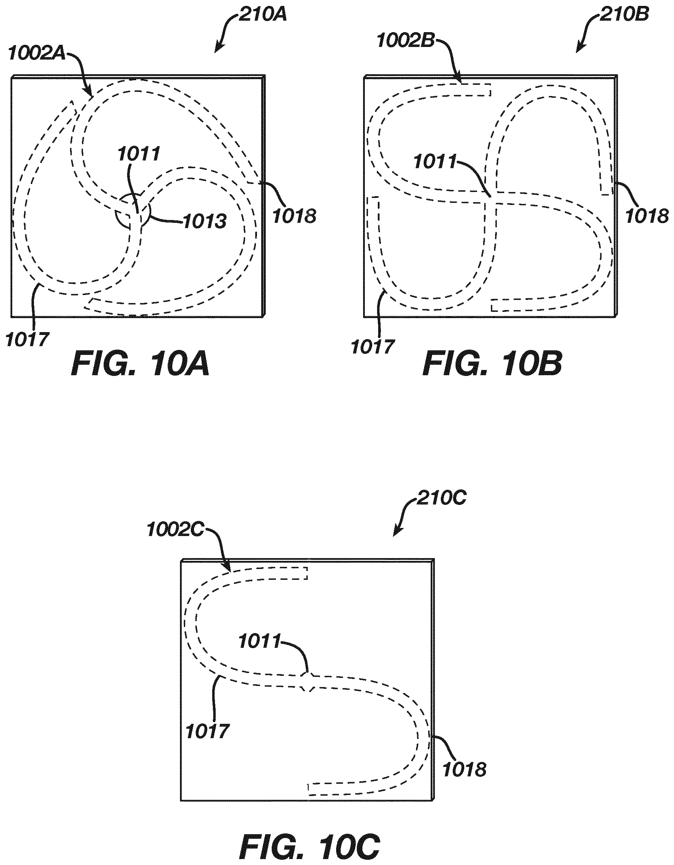

FIGs. 10A through 11B are schematic pictorial illustrations of cutting patterns for various C-shaped spines patterns 1002A-1002C and 1102A, 1102B from a planar sheet of material 210A-210E. As described supra, planar sheet of material 210 can cut to include a number of spines 214 ranging from about two to about five spines. As illustrated in FIGS. 10A through 10C, planar sheet of material 210 can include central intersection 1011 and respective spine patterns 1002A, 1002B, 1002C, which each include one or both of longitudinal scores 1017 and transverse scores 1018. In any of the embodiments disclosed herein, planar sheet of material 210 can also include a central intersection 1011 and spine patterns 1002 including an equiangular pattern 1013. Planar sheet of material 210 can include spine patterns including a number of spine patterns 1002 forming spines 214 in basket assembly 38. As would be understood by one of skill in the art, adjusting the number of spine patterns 1002 may impact a number of factors including, without limitation, stability, flexibility, surface contact, and ablation capacity of medical probe 22. FIGs. 11A and 11B provide example spine patterns 1102A, 1102B, which each include one or both of longitudinal scores 1117 and transverse scores 1118, and also include one or more cutout patterns 1112B, 1112B to form one or more cutouts 1112A, 1112B on central intersection 1111. In each of these examples provided, planar sheet of material 210 may also include central intersections 1111 and spine patterns including equiangular patterns 1113.

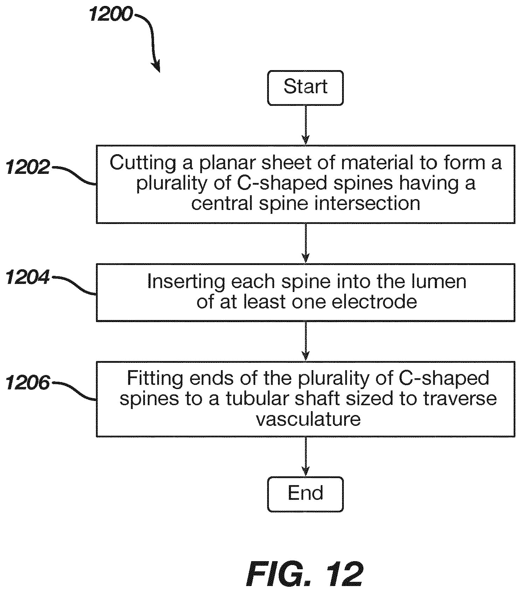

FIG. 12 is a flowchart illustrating a method 1200 of manufacturing a basket assembly 38, in accordance with an embodiment of the present invention. Method 1200 can include cutting 1202 a planar sheet of material 210 to form a plurality of C-shaped spines 214 including a central spine intersection 211. Cutting the plurality of C-shaped spines 214 can include cutting from a pattern 1002, 1102 including longitudinal and transverse scores 1018, 1018 from a planar sheet of resilient material. The planar sheet of resilient material can include shape-memory alloy such as nickel-titanium (also known as Nitinol), cobalt chromium, or any other suitable material. Method 1200 can include inserting 1204 each spine into a lumen of at least one electrode. The electrodes can be positioned such that the electrodes are offset between electrodes on adjacent spines. Method 1200 can include fitting 1206 ends of the plurality of C-shaped spines to a tubular shaft sized to traverse vasculature such that the central spine intersection is positioned at a distal end of the medical probe and respective spines are movable from a tubular configuration to a bowed configuration. As will be appreciated by one of skill in the art including the benefit of this disclosure, fitting 1206 an end of the spine into a tubular shaft can include attaching the spine 214 to a spine retention hub 90. Furthermore, the spine retention hub 90 and/or the spine 214 and the tubular shaft 84 can be inserted into a flexible insertion tube 30 to form the medical probe 22. Method 1200 can end at step 1206 or can also further include cutting a discrete cutout 212 at the central spine intersection 211. As described supra, the discrete cutout 212 can be a single cutout or two or more cutouts. In addition, the one or more discrete cutouts can be cut in a pattern to extend along at least a portion of each spine. In some examples, method 1200 can also include forming an approximately spheroid or oblate-spheroid shape with the C-shaped spines. Method 1200 can also include disposing an insulative sleeve over the C-shaped spines and within the lumen of the respective electrode.

As will be appreciated by one skilled in the art, method 1200 can include any of the various features of the disclosed technology described herein and can be varied depending on the particular configuration. Thus, method 1200 should not be construed as limited to the particular steps and order of steps explicitly described herein.

The embodiments or examples described above are cited by way of example, and the present invention is not limited by what has been particularly shown and described hereinabove. Rather, the scope of the invention is defined by the appended claims.

## Claims

1. A medical probe (22), comprising:
a tubular shaft (84) including a proximal end and a distal end and extending along a longitudinal axis (86);
an expandable basket assembly (38) proximate the distal end of the tubular shaft (84), the basket assembly (38) comprising two or more C-shaped spines, the C-shaped spines (214) converging at a central spine intersection (211) configured to permit a bending of the C-shaped spines (214), each C-shaped spine (214) comprising a respective end (216) connected to the distal end of the tubular shaft (84), the central spine intersection (211) being positioned on the longitudinal axis (86) at a distal end of the basket assembly; and
one or more electrodes (40, 740) coupled to each of the C-shaped spines (214), each electrode (40, 740) defining a lumen (770) through the electrode (40, 740) so that the C-shaped spine (214) extends through the lumen (770) of each of the one or more electrodes (40, 740),
**characterised in that** the expandable basket assembly is produced by cutting a planar sheet of material to form a plurality of C-shaped spines (214) including a central spine intersection, wherein the C-shaped spines are C-shaped in the plane of the planar sheet of material.

2. The medical probe (22) according to claim 1, wherein the two or more C-shaped spines (214) extend from the central spine intersection (211) in an equiangular pattern such that respective angles between respectively adjacent C-shaped spines (214) are approximately equal.

3. The medical probe (22) according to claim 1 or claim 2, wherein the expandable basket assembly (38) comprises two, three, or four C-shaped spines (214).

4. The medical probe (22) according to any preceding claim, wherein the planar sheet of material comprises nitinol.

5. The medical probe (22) according to any preceding claim, wherein the expandable basket assembly (38) further comprises one or more cutouts located at the central spine intersection (211).

6. The medical probe (22) according to claim 5, wherein the expandable basket assembly (38) comprises (i) a single cutout located at the central spine intersection (211), or (ii) at least one discrete cutout located proximate the central spine intersection (211) for each C-shaped spine (214).

7. The medical probe (22) according to claim 5 or claim 6, wherein the one or more cutouts extend along at least a portion of each C-shaped spine.

8. The medical probe (22) according to any preceding claim, further comprising a spine retention hub disposed proximate the distal end of the tubular shaft (84), the spine retention hub comprising:
a cylindrical member including a plurality of relief lands disposed on an outer surface of the cylindrical member to allow the respective end (216) of each C-shaped spine to be fitted into a relief land and retained therein, and
at least one electrode (40, 740) disposed at a distal portion of the spine retention hub.

9. The medical probe (22) according to any preceding claim, wherein each electrode (40, 740) comprises a wire relief adjacent the lumen (770) to allow for one or more wires to extend adjacent to the lumen (770).

10. The medical probe (22) according to any preceding claim, further comprising irrigation openings disposed proximate the distal end of the tubular shaft (84), the irrigation openings configured to deliver an irrigation fluid to the one or more electrodes (40, 740).

11. The medical probe (22) according to any preceding claim, further comprising a plurality of insulative sleeves each disposed over the respective C-shaped spine and within the lumen (770) of the respective electrode (40, 740).

12. The medical probe (22) according to any preceding claim, further comprising:
a plurality of wires each electrically joined to a respective electrode (40, 740) of the one or more of electrodes (40, 740).

13. The medical probe (22) according to claim 12, wherein at least a portion of the wires of the plurality of the wires respectively comprises (i) an electrically conductive core material comprising a first electrical conductivity, an electrically conductive cover material comprising a second electrical conductivity less than the first electrical conductivity, the electrically conductive cover material circumscribing the electrically conductive core material, and an insulative jacket circumscribing the electrically conductive cover material, or (ii) a plurality of strands and an insulative jacket circumscribing the plurality of the strands, and wherein each strand of the plurality of strands respectively comprises an electrically conductive core material comprising a first electrical conductivity and an electrically conductive cover material comprising a second electrical conductivity less than the first electrical conductivity, the electrically conductive cover material circumscribing the electrically conductive core material.

14. A method of constructing the medical probe (22) of any preceding claim, the method comprising:
cutting the planar sheet of material to form the plurality of C-shaped spines (214) converging at the central spine intersection;
inserting each C-shaped spine (214) into the lumen (770) of at least one electrode (40, 740); and
fitting ends of the plurality of C-shaped spines (214) to the tubular shaft (84) sized to traverse vasculature such that the central spine intersection is positioned at a distal end of the medical probe (22) and respective C-shaped spines (214) are movable from a tubular configuration to a bowed configuration.

15. The method according to claim 14, further comprising cutting the plurality of C-shaped spines (214) from a pattern comprising longitudinal and transverse scores.

16. The method according to claim 14 or claim 15, further comprising cutting a discrete cutout at the central spine intersection (211).

17. The method according to claim 16, further comprising cutting the discrete cutout along at least a portion of each C-shaped spine.

## Patentansprüche

1. Medizinische Sonde (22), umfassend:
einen rohrförmigen Schaft (84), der ein proximales Ende und ein distales Ende einschließt und sich entlang einer Längsachse (86) erstreckt;
eine expandierbare Korbanordnung (38) in der Nähe des distalen Endes des rohrförmigen Schafts (84), wobei die Korbanordnung (38) zwei oder mehr C-förmige Streben umfasst, wobei die C-förmigen Streben (214) an einem zentralen Strebenschnittpunkt (211) zusammenlaufen, der konfiguriert ist, um ein Biegen der C-förmigen Streben (214) zu ermöglichen, wobei jede C-förmige Strebe (214) ein jeweiliges Ende (216) umfasst, das mit dem distalen Ende des rohrförmigen Schafts (84) verbunden ist, wobei der zentrale Strebenschnittpunkt (211) auf der Längsachse (86) an einem distalen Ende der Korbanordnung positioniert ist; und
eine oder mehrere Elektroden (40, 740), die mit jeder der C-förmigen Streben (214) gekoppelt sind, wobei jede Elektrode (40, 740) ein Lumen (770) durch die Elektrode (40, 740) definiert, so dass sich die C-förmige Strebe (214) durch das Lumen (770) jeder der einen oder mehreren Elektroden (40, 740) erstreckt,
**dadurch gekennzeichnet, dass** die expandierbare Korbanordnung durch Schneiden einer ebenen Materialplatte hergestellt wird, um eine Vielzahl von C-förmigen Streben (214) zu bilden, die einen zentralen Strebenschnittpunkt einschließen, wobei die C-förmigen Streben in der Ebene der ebenen Materialplatte C-förmig sind.

2. Medizinische Sonde (22) nach Anspruch 1, wobei sich die zwei oder mehr C-förmigen Streben (214) von dem zentralen Strebenschnittpunkt (211) in einem gleichwinkligen Muster derart erstrecken, dass die jeweiligen Winkel zwischen jeweils benachbarten C-förmigen Streben (214) etwa gleich sind.

3. Medizinische Sonde (22) nach Anspruch 1 oder Anspruch 2, wobei die expandierbare Korbanordnung (38) zwei, drei oder vier C-förmige Streben (214) umfasst.

4. Medizinische Sonde (22) nach einem der vorstehenden Ansprüche, wobei die ebene Materialplatte Nitinol umfasst.

5. Medizinische Sonde (22) nach einem der vorstehenden Ansprüche, wobei die expandierbare Korbanordnung (38) ferner einen oder mehrere Ausschnitte umfasst, die sich an dem zentralen Strebenschnittpunkt (211) befinden.

6. Medizinische Sonde (22) nach Anspruch 5, wobei die expandierbare Korbanordnung (38) (i) eine einzelne Aussparung an dem zentralen Strebenschnittpunkt (211) oder (ii) mindestens eine diskrete Aussparung in der Nähe des zentralen Strebenschnittpunkts (211) für jede C-förmigen Strebe (214) umfasst.

7. Medizinische Sonde (22) nach Anspruch 5 oder Anspruch 6, wobei sich die eine oder die mehreren Aussparungen entlang mindestens eines Abschnitts jeder C-förmigen Strebe erstrecken.

8. Medizinische Sonde (22) nach einem der vorstehenden Ansprüche, ferner umfassend eine Strebenrückhaltenabe, die proximal zum distalen Ende des rohrförmigen Schafts (84) angeordnet ist, wobei die Strebenrückhaltenabe umfasst:
ein zylindrisches Element, einschließlich einer Vielzahl von Entlastungsflächen, die auf einer Außenoberfläche des zylindrischen Elements angeordnet sind, um zu ermöglichen, dass das jeweilige Ende (216) jeder C-förmigen Streben in eine Entlastungsfläche eingepasst und darin gehalten wird, und
mindestens eine Elektrode (40, 740), die an einem distalen Abschnitt der Strebenrückhaltenabe angeordnet ist.

9. Medizinische Sonde (22) nach einem der vorstehenden Ansprüche, wobei jede Elektrode (40, 740) eine Drahtentlastung benachbart zu dem Lumen (770) umfasst, um zu ermöglichen, dass sich ein oder mehrere Drähte benachbart zu dem Lumen (770) erstrecken.

10. Medizinische Sonde (22) nach einem der vorstehenden Ansprüche, ferner umfassend Spülöffnungen, die in der Nähe des distalen Endes des rohrförmigen Schafts (84) angeordnet sind, wobei die Spülöffnungen konfiguriert sind, um ein Spülfluid an die eine oder mehreren Elektroden (40, 740) abzugeben.

11. Medizinische Sonde (22) nach einem der vorstehenden Ansprüche, ferner umfassend eine Vielzahl von Isolierhüllen, die jeweils über der jeweiligen C-förmigen Strebe und innerhalb des Lumens (770) der jeweiligen Elektrode (40, 740) angeordnet sind.

12. Medizinische Sonde (22) nach einem der vorstehenden Ansprüche, ferner umfassend:
eine Vielzahl von Drähten, die jeweils an eine jeweilige Elektrode (40, 740) der einen oder mehreren Elektroden (40, 740) elektrisch angeschlossen sind.

13. Medizinische Sonde (22) nach Anspruch 12, wobei mindestens ein Abschnitt der Drähte der Vielzahl von Drähten jeweils (i) ein elektrisch leitfähiges Kernmaterial, umfassend eine erste elektrische Leitfähigkeit, ein elektrisch leitfähiges Ummantelungsmaterial, umfassend eine zweite elektrische Leitfähigkeit, die geringer als die erste elektrische Leitfähigkeit ist, wobei das elektrisch leitfähige Ummantelungsmaterial das elektrisch leitfähige Kernmaterial umgibt, und eine isolierende Ummantelung, die das elektrisch leitfähige Ummantelungsmaterial umgibt, oder (ii) eine Vielzahl von Litzen und eine Isolierhaube, die die Vielzahl von Litzen umgibt, umfasst, und wobei jede Litze der Vielzahl von Litzen jeweils ein elektrisch leitfähiges Kernmaterial, umfassend eine erste elektrische Leitfähigkeit, und ein elektrisch leitfähiges Ummantelungsmaterial, umfassend eine zweite elektrische Leitfähigkeit, die geringer als die erste elektrische Leitfähigkeit ist, umfasst, wobei das elektrisch leitfähige Ummantelungsmaterial das elektrisch leitfähige Kernmaterial umgibt.

14. Verfahren zum Herstellen der medizinischen Sonde (22) nach einem der vorstehenden Ansprüche, das Verfahren umfassend:
Schneiden der ebenen Materialplatte, um die Vielzahl von C-förmigen Streben (214) zu bilden, die an dem zentralen Strebenschnittpunkt zusammenlaufen;
Einführen jeder C-förmigen Strebe (214) in das Lumen (770) mindestens einer Elektrode (40, 740); und
Anbringen von Enden der C-förmigen Streben (214) an den rohrförmigen Schaft (84), der bemessen ist, um ein Gefäßsystem derart zu durchqueren, dass der zentrale Strebenschnittpunkt an einem distalen Ende der medizinischen Sonde (22) positioniert ist und die jeweiligen C-förmigen Streben (214) von einer rohrförmigen Konfiguration in eine gebogene Konfiguration bewegbar sind.

15. Verfahren nach Anspruch 14, ferner umfassend das Schneiden der Vielzahl von C-förmigen Streben (214) aus einem Muster, das Längs- und Querkerben umfasst.

16. Verfahren nach Anspruch 14 oder 15, ferner umfassend das Schneiden eines diskreten Ausschnitts an dem zentralen Strebenschnittpunkt (211).

17. Verfahren nach Anspruch 16, ferner umfassend das Schneiden des diskreten Ausschnitts entlang mindestens eines Abschnitts jeder C-förmigen Strebe.

## Revendications

1. Sonde médicale (22), comprenant :
un arbre tubulaire (84) comportant une extrémité proximale et une extrémité distale, et s'étendant le long d'un axe longitudinal (86) ;
un ensemble panier (38) expansible à proximité de l'extrémité distale de l'arbre tubulaire (84), l'ensemble panier (38) comprenant deux arceaux en forme de C ou plus, les arceaux en forme de C (214) convergeant au niveau d'une intersection d'arceaux centrale (211) conçue pour permettre une courbure des arceaux en forme de C (214), chaque arceau en forme de C (214) comprenant une extrémité respective (216) reliée à l'extrémité distale de l'arbre tubulaire (84), l'intersection d'arceaux centrale (211) étant positionnée sur l'axe longitudinal (86) au niveau d'une extrémité distale de l'ensemble panier ; et
une ou plusieurs électrodes (40, 740) accouplées à chacune des arceaux en forme de C (214), chaque électrode (40, 740) définissant une lumière (770) à travers l'électrode (40, 740) de sorte que l'arceau en forme de C (214) s'étend à travers la lumière (770) de chacune de la ou des électrodes (40, 740),
**caractérisée en ce que** l'ensemble panier expansible est produit en découpant une feuille plane de matériau pour former une pluralité d'arceaux en forme de C (214) comportant une intersection d'arceaux centrale, dans laquelle les arceaux en forme de C sont en forme de C dans le plan de la feuille plane de matériau.

2. Sonde médicale (22) selon la revendication 1, dans laquelle les deux arceaux en forme de C (214) ou plus s'étendent à partir de l'intersection d'arceaux centrale (211) selon un motif équiangulaire de telle sorte que des angles respectifs entre des arceaux en forme de C (214) respectivement adjacents sont approximativement égaux.

3. Sonde médicale (22) selon la revendication 1 ou la revendication 2, dans laquelle l'ensemble panier (38) expansible comprend deux, trois ou quatre arceaux en forme de C (214).

4. Sonde médicale (22) selon l'une quelconque revendication précédente, dans laquelle la feuille plane de matériau comprend du nitinol.

5. Sonde médicale (22) selon l'une quelconque revendication précédente, dans laquelle l'ensemble panier (38) expansible comprend en outre une ou plusieurs découpes situées au niveau de l'intersection d'arceaux centrale (211).

6. Sonde médicale (22) selon la revendication 5, dans laquelle l'ensemble panier (38) expansible comprend (i) une découpe unique située au niveau de l'intersection d'arceaux centrale (211), ou (ii) au moins une découpe discrète située à proximité de l'intersection d'arceaux centrale (211) pour chaque arceau en forme de C (214).

7. Sonde médicale (22) selon la revendication 5 ou la revendication 6, dans laquelle la ou les découpes s'étendent le long d'au moins une partie de chaque arceau en forme de C.

8. Sonde médicale (22) selon l'une quelconque revendication précédente, comprenant en outre un moyeu de retenue d'arceau disposé à proximité de l'extrémité distale de l'arbre tubulaire (84), le moyeu de retenue d'arceau comprenant :
un élément cylindrique comportant une pluralité de zones de dégagement disposées sur une surface externe de l'élément cylindrique pour permettre à l'extrémité respective (216) de chaque arceau en forme de C d'être ajustée dans la zone de dégagement et retenue dans celle-ci, et
au moins une électrode (40, 740) disposée au niveau d'une partie distale du moyeu de retenue d'arceau.

9. Sonde médicale (22) selon l'une quelconque revendication précédente, dans laquelle chaque électrode (40, 740) comprend un dégagement de fil adjacent à la lumière (770) pour permettre à un ou plusieurs fils de s'étendre de manière adjacente à la lumière (770).

10. Sonde médicale (22) selon l'une quelconque revendication précédente, comprenant en outre des ouvertures d'irrigation disposées à proximité de l'extrémité distale de l'arbre tubulaire (84), les ouvertures d'irrigation étant conçues pour délivrer un fluide d'irrigation à la ou aux électrodes (40, 740).

11. Sonde médicale (22) selon l'une quelconque revendication précédente, comprenant en outre une pluralité de manchons isolants disposés chacun sur l'arceau en forme de C respectif et à l'intérieur de la lumière (770) de l'électrode (40, 740) respective.

12. Sonde médicale (22) selon l'une quelconque revendication précédente, comprenant en outre :
une pluralité de fils, chacun relié électriquement à une électrode (40, 740) respective de la ou des électrodes (40, 740).

13. Sonde médicale (22) selon la revendication 12, dans laquelle au moins une partie des fils de la pluralité des fils comprend respectivement (i) un matériau de noyau électriquement conducteur comprenant une première conductivité électrique, un matériau de revêtement électriquement conducteur comprenant une seconde conductivité électrique inférieure à la première conductivité électrique, le matériau de revêtement électriquement conducteur entourant le matériau de noyau électriquement conducteur, et une enveloppe isolante entourant le matériau de revêtement électriquement conducteur, ou (ii) une pluralité de brins et une enveloppe isolante entourant la pluralité de brins, et dans laquelle chaque brin de la pluralité de brins comprend respectivement un matériau de noyau électriquement conducteur comprenant une première conductivité électrique et un matériau de revêtement électriquement conducteur comprenant une seconde conductivité électrique inférieure à la première conductivité électrique, le matériau de revêtement électriquement conducteur entourant le matériau de noyau électriquement conducteur.

14. Procédé permettant de fabriquer une sonde médicale (22) selon l'une quelconque revendication précédente, le procédé comprenant :
la découpe de la feuille plane de matériau pour former la pluralité d'arceaux en forme de C (214) convergeant au niveau de l'intersection d'arceaux centrale ;
l'insertion de chaque arceau en forme de C (214) dans la lumière (770) d'au moins une électrode (40, 740) ; et
l'ajustement d'extrémités de la pluralité d'arceaux en forme de C (214) à l'arbre tubulaire (84) dimensionné pour traverser un système vasculaire de telle sorte que l'intersection d'arceaux centrale est positionnée au niveau d'une extrémité distale de la sonde médicale (22) et que des arceaux en forme de C (214) respectifs sont mobiles d'une configuration tubulaire à une configuration arquée.

15. Procédé selon la revendication 14, comprenant en outre la découpe de la pluralité d'arceaux en forme de C (214) d'un motif comprenant des rainures longitudinales et transversales.

16. Procédé selon la revendication 14 ou la revendication 15, comprenant en outre la découpe d'une découpe discrète au niveau de l'intersection d'arceaux centrale (211).

17. Procédé selon la revendication 16, comprenant en outre la découpe d'une découpe discrète le long d'au moins une partie de chaque arceau en forme de C.
